# FASCICULE DE BREVET EUROPEEN

(11) **EP 2 713 929 B1**
(45) Date de publication et mention de la délivrance du brevet: **15.11.2017**
(21) Numéro de dépôt: 12728721.7
(22) Date de dépôt: 22.05.2012
(51) Int. Cl.: A61B 90/90, A61B 17/00

(54) **SYSTEME ET PROCEDE DE TRACABILITE D'UN SYSTEME D'INSTRUMENTATION CHIRURGICALE**
SYSTEM UND METHODE ZUR RÜCKVERFOLGUNG EINES CHIRURGISCHEN INSTRUMENTATIONS-SYSTEMS
SYSTEM AND METHOD OF TRACEABILITY FOR A SURGICAL INSTRUMENT SYSTEM

(30) Priorité: 01.06.2011 FR 1101703
(43) Date de publication de la demande: 09.04.2014
(73) Titulaire: Safe Orthopaedics, 95610 Eragny-sur-Oise (FR)
(72) Inventeur: DUMOUCHEL, Pierre, 92600 Asnieres Sur Seine (FR)
(74) Mandataire: IP Trust
(86) Numéro de dépôt international: PCT/FR2012/051139
(87) Numéro de publication internationale: WO 2012/164201

(56) Documents cités:
- EP-A2- 1 855 264
- WO-A1-2005/027767
- WO-A1-2011/035277
- WO-A2-03/081379
- DE-A1- 4 420 707
- US-A1- 2008 077 444

## Description

### Domaine de l'invention

La présente invention concerne le domaine de la gestion d'instruments et d'implants chirurgicaux à usage unique, et en particulier de prothèses orthopédiques et de l'instrumentation associée.

### Etat de la technique

On connaît dans l'état de la technique la demande de brevet américaine US2003/0182299 décrivant un système constitué par une pluralité de kits d'instruments stériles, destinés à la pose d'implants non compris dans les kits. Chaque kit est marqué avec un identifiant unique.

On connaît aussi la demande de brevet WO03081379 décrivant une solution de gestion d'un kit unique contenant des instruments chirurgicaux et des implants intra-oculaires.

Cette solution ne permet pas d'assurer la traçabilité de systèmes chirurgicaux où l'instrumentation est fournie dans un premier kit, et une partie des implants dans un autre kit distinct du premier.

On connaît encore les documents DE4420707, EP1855264, WO2005027767 et WO2011035277 décrivant étiquettes et procédés de traçabilité applicables dans le domaine médical.

On connaît également la demande de brevet internationale WO2008/043921 décrivant un procédé et un suivi de produits médicaux dans lequel on marque et on identifie le produit et/ou son conditionnement avec une première référence. Cette première référence est stockée dans un premier fichier. Le produit est livré sous une forme conditionnée en vue de son utilisation ultérieure par le client, pour le traitement d'un patient répertorié dans un deuxième fichier. On détecte automatiquement et à distance les références du produit lors de sa livraison. Ces références sont stockées dans un troisième fichier de gestion du stock du client lors d'une opération. La solution de l'art antérieur prévoit de détecter automatiquement la sortie du produit du stock, d'insérer les références du produit dans le deuxième fichier correspondant au patient pour lequel il est - ou va être - utilisé. On constitue ensuite un quatrième fichier comprenant les références partielles provenant du fichier du patient et les références déterminées du produit pour traçage et on transmet automatiquement ou semi-automatiquement ce quatrième fichier au fournisseur du produit. On connaît de la demande US 2008/077444 un système de traçabilité chirurgicale comprenant un support destiné à recevoir des informations par rapport à l'opération à effectuer.

### Inconvénients des solutions de l'art antérieur

Les solutions proposées dans l'art antérieur présentent plusieurs inconvénients techniques.

En premier lieu, elles nécessitent impérativement l'accès à un système d'information numérique pour le traitement et l'exploitation des données d'identification.

Pour une utilisation en bloc opératoire, l'utilisation d'un moyen informatique n'est pas appropriée, et cela conduit à un enregistrement des informations avant ou après l'intervention. Le fait de différer cet enregistrement est source d'erreurs ou d'oublis, ce qui est préjudiciable à la confiance dans la traçabilité des produits et instruments utilisés.

En deuxième lieu, les solutions de l'art antérieur prévoient, dans le cas de la demande WO2008/043921, l'enregistrement dans un système d'information de l'identification du patient. Cette contrainte se heurte aux règles régissant la confidentialité des données médicales et de leur gestion, même si la description de cette demande évoque la gestion secrète de l'identification du patient.

La solution proposée dans la demande US2003/0182299 ne prévoit au contraire aucun lien entre le patient et les matériels chirurgicaux et concerne uniquement la gestion des stocks par un système informatique.

Aucune des solutions de l'art antérieur ne permet donc d'assurer une traçabilité complète par l'hôpital respectant totalement les règles relatives à l'anonymat du patient. Cette lacune est problématique car les personnes concernées par cette gestion des systèmes d'instrumentation chirurgicale ne sont pas tous des médecins liées par le secret médical.

Par ailleurs, aucune des solutions de l'art antérieur ne permet d'assurer la traçabilité de l'ensemble des instruments et implants utilisés pour un patient déterminé.

### Solution apportée par l'invention

La présente invention a pour objet de remédier à ces inconvénients en proposant un système simple dans sa mise en oeuvre, et susceptible d'être exploités dans le cadre strict de la gestion des données médicaux-légales.

A cet effet, l'invention concerne selon son acception la plus générale un système d'instrumentation chirurgicale constitué par un kit d'instruments à usage unique de pose d'implant et au moins un kit d'implants stériles, chacun desdits kits comprenant un identifiant caractérisé en ce que au moins l'un desdits kits contient un identifiant unique, ainsi qu'un support d'enregistrement d'identifiants additionnels,
- l'un desdits identifiants additionnels étant un code du patient, collecté lors de l'utilisation dudit kit,
- au moins un autre identifiant additionnel étant l'identifiant d'au moins un kit complémentaire comportant un identifiant transférable sur ledit support d'enregistrement d'identifiants additionnels, au moment de l'utilisation dudit kit complémentaire.

Un « kit » peut comporter un ou plusieurs instruments, ou un et plusieurs implants, ou encore un ou plusieurs instruments et un ou plusieurs implants.

Selon des variantes, ledit identifiant unique est constitué par un code reconnaissable optiquement ou est enregistré dans une mémoire électronique.

Selon un premier mode de mise en oeuvre avantageux, le support d'enregistrement d'identifiants additionnels est constitué par une feuille portant ledit identifiant unique ainsi que des zones pour recevoir les supports des identifiants transférables et une zone pour recevoir le code (8) du patient.

Selon un deuxième mode de mise en oeuvre ne faisant pas partie de l'invention revendiquée, le support d'enregistrement des identifiants additionnels est constitué par un fichier informatique apte à recevoir ledit identifiant unique, le code du patient ainsi que les supports des identifiants transférables.

De préférence, le code du patient est anonyme et non-unique.

Selon une variante, le système selon l'invention comporte en outre une base de données pour l'enregistrement des informations relatives à la fabrication, au conditionnement et à la stérilisation de l'ensemble des kits, le système comprenant des moyens pour rapprocher les informations issues du support d'enregistrement d'identifiants additionnels à partir de l'identifiant unique associé audits support.

Avantageusement, le kit comportant ledit identifiant unique et ledit support d'enregistrement est le kit d'instruments de pose.

L'invention concerne également un procédé de traçabilité d'un système d'instrumentation chirurgicale constitué par un kit d'instruments à usage unique de pose d'implant et au moins un kit d'implants stériles, chacun desdits kits comprenant un identifiant caractérisé en ce que l'on enregistre un code du patient sur un support d'enregistrement d'identifiants additionnels associé à l'un desdits kits comprenant un identifiant unique, et en ce que l'on enregistre en outre sur ledit support d'enregistrement d'identifiants additionnels au moins un identifiant transférable d'au moins un kit complémentaire, au moment de l'utilisation dudit kit complémentaire.

Selon une variante, le procédé comporte une étape de transmission, à une base de donnée de collecte, dudit supports d'enregistrement à l'issue de l'exploitation dudit système sur un patient, et en ce qu'il comporte une étape d'édition d'une fiche de synthèse à partir des données collectées au moins.

Selon une autre variante, le procédé comporte une étape de transmission à un collecteur de déchets à recycler, une fiche de traçabilité regroupant les informations provenant de la fiche d'enregistrement.

### Description détaillée d'un exemple de l'invention

La présente invention sera mieux comprise à la lecture de la description qui suit, se référant à des exemples non limitatifs de mise en oeuvre illustrés par les dessins annexés où :

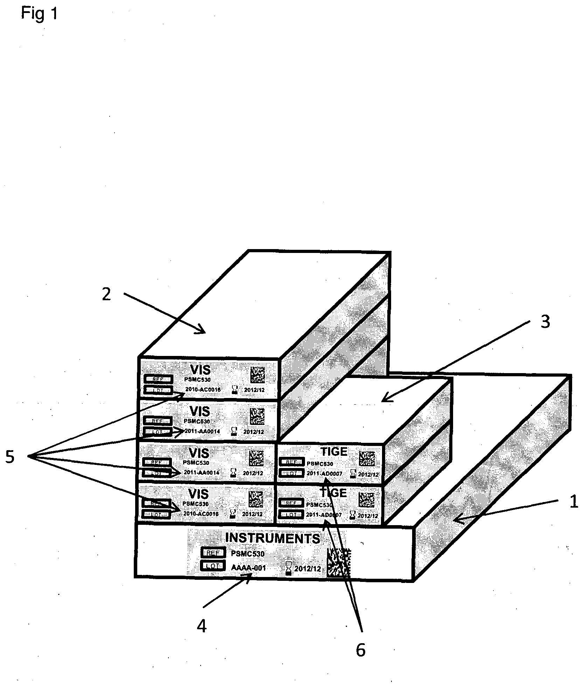

- la figure 1 représente une vue schématique de kits pour la mise en oeuvre de l'invention
- Les figures 2 et 2bis représentent des vues d'un kit après ouverture ainsi que les identifiants associés
- la figure 3 représente un exemple de support d'enregistrement d'identifiants additionnels
- La figure 4 représente une vue des flux d'informations lors de la mise en oeuvre de l'invention

### Description détaillée d'un exemple de réalisation

Le système selon l'invention est composé de plusieurs kits à usage unique, un de ces kits comprenant un ou plusieurs instruments de pose conditionnés de manière stérile, un autre de ces kits comprenant un ou plusieurs implants ou prothèses stériles, destinés à être posés avec les instruments du premier kit.

### Contexte de l'utilisation de kits à usage unique

L'état de l'art dans le domaine de la chirurgie lombaire de la colonne vertébrale consiste à mettre à disposition des chirurgiens des implants, et des sets d'instruments. Certaines sociétés spécialisées du domaine proposent des implants conditionnés de manière stériles, prêts à l'emploi.

Il est également connu d'utiliser des sets d'instruments réutilisables à volonté après décontamination et stérilisation, ce qui conduit à de nombreux désavantages :
- Le risque de contamination d'un patient à un autre est très élevé, le nettoyage,
- La décontamination et la stérilisation sont des étapes qui sont très difficiles à réaliser correctement à la vue des formes complexes, avec de nombreuses cavités sur les instruments.
- Ces étapes sont à la charges des hôpitaux est représentent des couts importants à la fois humain et matériel.

La perte, la casse, l'usure, la détérioration d'un instrument peut entrainer des conséquences désastreuses pour le patient suivant, voir engendrer l'annulation de la chirurgie. La logistique comporte également de nombreux désavantages, lourde et couteuse à la fois pour l'industriel et l'hôpital, elle implique le plus souvent des inventaires importants.

Les rotations de kits entre l'hôpital et le service logistique de l'industriel sont donc très nombreuses, ce qui augmente significativement les risques de pertes et d'erreurs. L'invention prend ainsi en compte l'aspect sécurité à la fois pour le patient et le chirurgien, ainsi que les aspects financiers, humain et matériel pour l'hôpital.

### Exemple de kits pour la chirurgie du rachis

L'invention est décrite dans ce qui suit en référence à un système de vis pédiculaire décrite dans la demande de brevet français FR09/06369.

Le système est composé de plusieurs kits à usage unique :
- Le kit instrument, constituant le « kit d'instruments à usage unique de pose d'implant (1) » comprend l'ensemble des instruments nécessaires à la préparation, l'ancrage de l'implant dans les vertèbres et le verrouillage du montage. Ce kit sécurise le geste du chirurgien par l'utilisation d'instruments neufs à chaque intervention, annule tous risques de contamination nosocomiale et permet une réduction significative des coûts de santé publique ;
- des kits d'implants stériles (2, 3) constitués par :
   - un kit implant (2) comprend la vis pédiculaire et son bouchon, pré-montés sur les instruments de pose rendant l'utilisation très intuitive. Ce kit est décliné en plusieurs références : variantes en diamètre et longueur de vis
   - Un kit (3) de tiges comprend deux tiges de liaison.

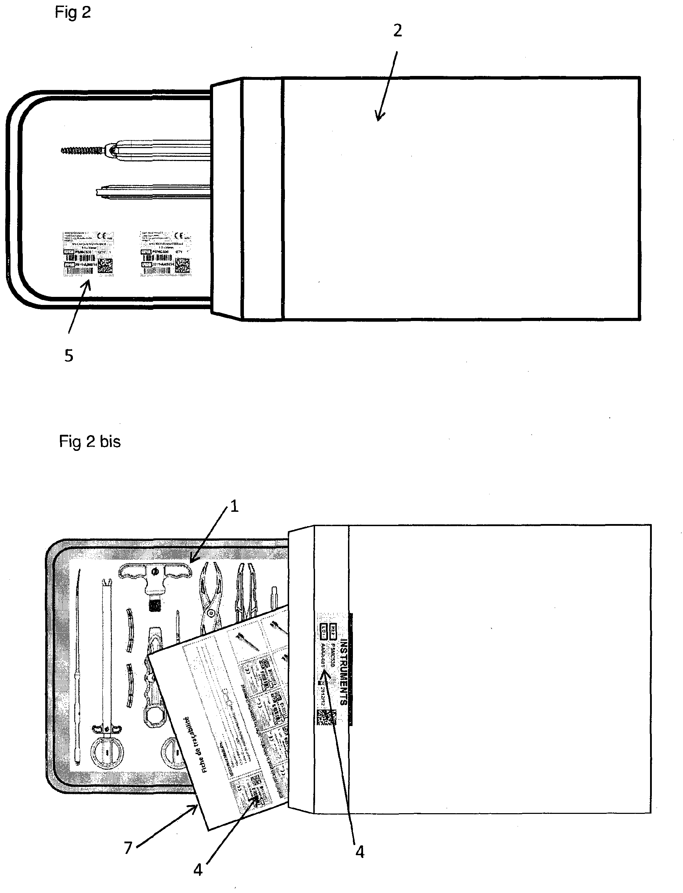

Les figures 2 et 2bis représentent des vues d'un kit respectivement d'implants (2) et d'instruments (1) après ouverture ainsi que les identifiants associés (4).

Les kits instruments (1) contiennent un identifiant unique (4) permettant lors de son utilisation au bloc opératoire de le relier à une chirurgie (dossier patient), et aux autres kits (2, 3) (tiges ou vis) utilisés. Cet identifiant unique (4) peut-être un numéro de lot sérialisé, un code barre du type code matriciel « data matrix », un code enregistré dans une mémoire électronique ou tout autre moyen...

Dans chaque kit implant (2, 3), conformément aux directives, plusieurs étiquettes transférables (5, 6) reprenant la traçabilité complète du contenu sont disponibles.

Un support d'enregistrement constitué par une fiche de suivi papier est fournie dans le kit instrument reprenant son identifiant unique (4), et permettant la mise en place de l'ensemble des étiquettes « implants » (5) utilisées lors de la chirurgie, ainsi qu'une codification du patient associé (Codification pour des raisons de confidentialité). Cette codification est de préférence de type « non unique », par exemple les trois premiers caractères du nom du patient, voire la date de l'intervention sans précision de l'heure. Cela garantie l'absence de risque d'atteinte au secret médical.

Les étiquettes (5) transférables sont constituées dans l'exemple décrit par des étiquettes autocollantes sur laquelle sont imprimés un identifiant, par exemple le numéro du lot de fabrication. Cet identifiant n'est pas nécessairement unique, mais il est obligatoirement spécifique à un même composant, issu de la même série de fabrication.

### Support d'enregistrement d'identifiants additionnels

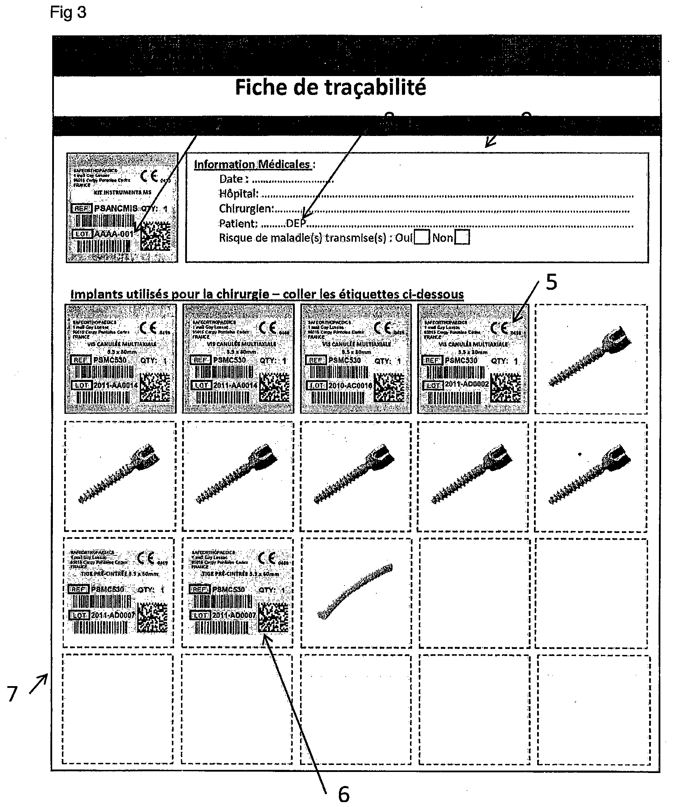

La figure 3 représente un exemple de support d'enregistrement d'identifiants additionnels (7).

Cette fiche de suivi (7), incluse dans un des kits, dans l'exemple décrit, dans le kit instrument (1), est utilisée d'une part par l'hôpital pour compléter le dossier patient, et d'autre part pour faciliter la facturation et le réapprovisionnement des kits et accessoirement permettre le recyclage des instruments à usage unique.

Elle présente des zones destinées au collage des étiquettes (5, 6) retirées des kits implants (2, 3) lors de l'utilisation de ceux-ci.

Sur cette fiche de suivi, y est complété, à titre d'exemple, afin de permettre :
- L'identification anonyme du patient, permettant à l'hôpital d'identifier le patient sans divulguer des informations confidentielles aux autres acteurs de l'approvisionnement et éventuellement du recylage des instruments à usage unique.
- La déclaration de risque maladie transmissible : l'hôpital déclare le risque de maladies transmissibles si le patient est porteur du HIV, de Creutzfeld Jacob...
- L'identifiant des autres kits consommés.

Cette fiche de suivi est envoyée à un opérateur renseignant une base de données. La base de donnée ainsi complétée peut déclencher la facturation, le réapprovisionnent, le recyclage des produits ainsi que la transmission d'une fiche de synthèse regroupant l'ensemble des informations de traçabilité, tant celle provenant de l'hôpital que celle provenant de la chaine de fabrication, de conditionnement et de stérilisation.

Cette fiche de suivi (7) peut-être directement complétée via la base de données interactive.

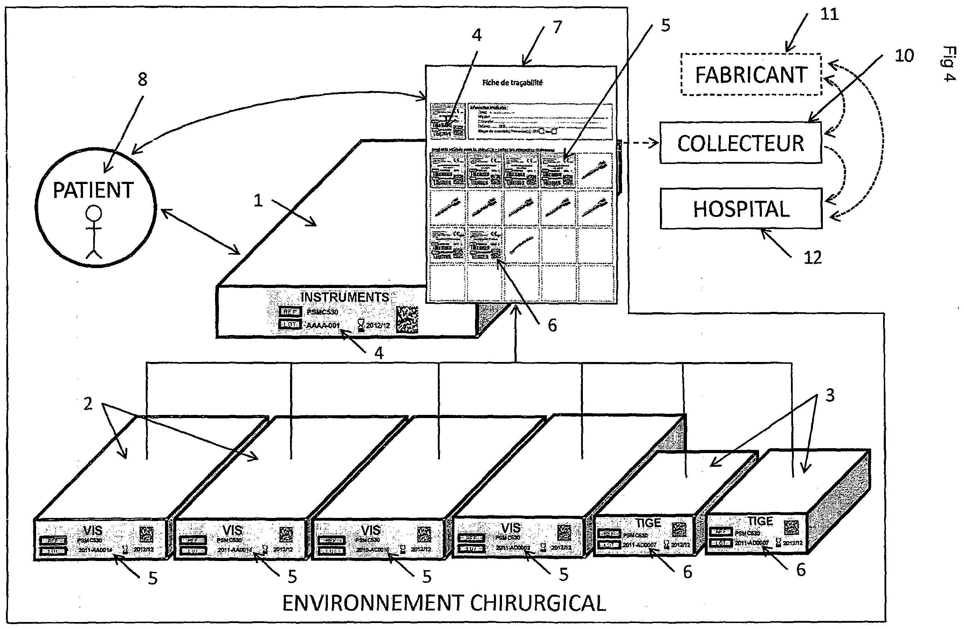

La figure 4 représente une vue schématique du flux d'informations.

L'élément de référence des informations est constitué par l'identifiant unique (4) associé au kit instrument (1), ainsi qu'au support d'enregistrement (7) contenu dans celui-ci.

Après l'ouverture du kit (1) pour une intervention sur un patient, on enregistre sur le support d'enregistrement (7) une information anonyme (8) et non directement liée au patient, par exemple par l'inscription dans la rubrique (9), d'un code (8). Ce code (8) peut être constitué par les trois premières lettres du nom du patient. Ce triplet n'est pas unique, puisque plusieurs noms de famille peuvent débuter avec la même chaine de caractères, par exemple le trigramme « DEP » désignant aussi bien «Claude DEPIPEAU» et que « Marcel DEPUY». Le code constitué par ce trigramme ne permet donc pas à lui seul l'identification du patient, et sa transmission n'entre pas dans le cadre des règles relatives au secret médical.

L'hôpital pourra seul reconstituer le lien entre le patient, son identité complète et la fiche de suivi (7). Pour cela, l'hôpital associera l'identifiant unique (4) provenant du kit instrument (1) avec le dossier patient en sa possession, ou enregistrera sur un duplicata de la fiche de suivi (7) l'identifiant unique du patient, duplicata qui restera sous le seul contrôle de l'hôpital.

En particulier, le kit d'instrumentation (1) peut comporter une étiquette autocollante sur lequel est imprimée l'identifiant unique ou un support transférable pour faciliter l'association de cet identifiant avec le dossier patient.

L'hôpital disposera ainsi :
- d'un dossier patient régi par le secret médical
- d'une fiche de suivi anonyme
- d'une information univoque formée par l'identifiant unique et le code non unique du patient, permettant de rapprocher le dossier patient avec la fiche de suivi (7).

Par contre, le fabricant ne dispose pas du dossier patient et le code patient apposé sur la fiche de suivi (7) ne lui permet en aucun cas d'identifier le patient concerné.

Lors de l'intervention, le chirurgien choisi parmi les kits d'implants (2, 3) celui ou ceux nécessairement pour l'opération chirurgicale en cours. Chacun de ces kits (2, 3) comporte des identifiants qui peuvent être communs à plusieurs kits de la même série. Par exemple, plusieurs kits de tiges comportent un même identifiant (XYZ) pour ceux d'une première série, et (MNO) pour ceux d'une deuxième série. Ces identifiants (5, 6) sont par ailleurs imprimés sur une étiquette autocollante contenue dans le conditionnement de l'implant.

L'étiquette du kit utilisé est détachée du kit et reportée sur la fiche de suivi (7).

A la fin de l'intervention, la fiche de suivi (7) est transmise à un collecteur (10), par exemple au distributeur ou au fabricant des kits par télécopie, sous forme de fichiers image numérique ou par courrier, ou encore par le biais d'un téléphone portable équipé d'un moyen de prise de vue.

Le collecteur (10) enregistre l'ensemble des informations de la fiche de suivi (7) et le cas échéant les rapproche des informations venant du fabricant (11), et incluant le cas échéant les données relatives au conditionnement et à la stérilisation.

Le collecteur peut ensuite retransmettre à l'hôpital (12) une fiche de traçabilité identifiée par la combinaison de l'identifiant unique et de le code anonyme (8) du patient, et comprenant des informations sur l'état de chacun des outils et implants correspondant aux identifiants enregistrés sur la feuille de suivi (7).

Cette fiche de traçabilité peut être ensuite associée par l'hôpital au dossier patient, qu'il est seul à être en mesure d'exploiter et de rapprocher, car l'hôpital est le seul à détenir les informations permettant d'associer le code non-unique (8) avec l'identifiant secret du patient, par l'intermédiaire de l'identifiant unique (4) du kit instrument.

### Variante pour la gestion du recyclage

A ce jour, les produits à usage unique communément utilisés sont traités comme des DASRI (Déchets d'activités de Soins à Risques Infections), jetés dans des poubelles spécifiques puis incinérés.

Les emballages des dispositifs médicaux selon l'invention sont conçus de manière à servir d'emballage pour le recyclage.

Après la chirurgie, l'hôpital déclare l'utilisation du dispositif en renseignant la fiche patient dans la base de données, qui comme décrit précédemment déclenche le réapprovisionnement, mais permet également de statuer sur la possibilité de recyclage :

Si le patient ne présente aucune maladie transmissible (HIV, Creutzfeld Jacob...), le dispositif est décontaminé par l'hôpital, reconditionné dans l'emballage. Le fabricant est identifié soit par un numéro de lot unique et/ou un marque (autocollant...), stocké jusqu'à la collecte organisé par le fabricant.

Parallèlement, ces produits sont inventoriés dans la base de données comme produits à retraiter, si le patient est malade (HIV, Creutzfeld Jacob...), présente des symptômes sans que le diagnostic soit fixé, que la traçabilité ne soit pas possible juste après la chirurgie, les produits sont incinérés selon les procédures de l'hôpital.

Le fabricant constitue des dossiers de recyclage en éditant à partir de la base de données qui regroupe les informations de production initiale et la fiche de suivi et organise la collecte des pièces. Les produits sont physiquement envoyés vers des sites de retraitement chargés du nettoyage, du tri des matériaux et de leurs revalorisations, quel quelles soient.

## Revendications

1. - Système de traçabilité d'un système d'instrumentation chirurgicale, le système étant constitué par un kit d'instruments à usage unique de pose d'implants (1) et au moins un kit d'implants stériles (2, 3), chacun desdits kits comprenant un identifiant, un premier des kits (2, 3) comportant un identifiant unique (4) permettant l'identification du kit comprenant l'identifiant unique, le deuxième kit (3, 2) comportant au moins un identifiant sur un support transférable, le premier kit comprenant un support (7) d'enregistrement d'identifiants additionnels constitué par une feuille portant un identifiant reprenant l'identifiant unique, ladite feuille étant contenue à l'intérieur du premier kit, l'un desdits identifiants additionnels pouvant être un code associé au patient, collecté lors de l'utilisation dudit kit,
au moins un autre identifiant additionnel étant l'identifiant sur support transférable (5, 6) du deuxième kit, ledit identifiant sur support transférable (5, 6) étant destiné à être transféré sur ledit support (7) d'enregistrement d'identifiants additionnels au moment de l'utilisation du deuxième kit.

2. - Système de traçabilité d'un système d'instrumentation chirurgicale selon la revendication 1 **caractérisé en ce que** ledit identifiant unique (4) est constitué par un code reconnaissable optiquement.

3. - Système de traçabilité d'un système d'instrumentation chirurgicale selon la revendication 1 **caractérisé en ce que** ledit identifiant unique (4) est constitué par un code enregistré dans une mémoire électronique.

4. - Système de traçabilité d'un système d'instrumentation chirurgicale selon l'une quelconque des revendications précédentes **caractérisé en ce que** le support d'enregistrement (7) d'identifiants additionnels comporte en outre des zones pour recevoir les supports des identifiants transférables et une zone pour recevoir le code du patient.

5. - Système de traçabilité d'un système d'instrumentation chirurgicale selon l'une quelconque des revendications précédentes **caractérisé en ce qu'**il comporte en outre une base de données pour l'enregistrement des informations relatives à la fabrication, au conditionnement et à la stérilisation de l'ensemble des kits, le système comprenant des moyens pour rapprocher les informations issues du support d'enregistrement (7) d'identifiants additionnels à partir de l'identifiant unique associé audit support.

6. - Système de traçabilité d'un système d'instrumentation chirurgicale selon l'une quelconque des revendications précédentes **caractérisé en ce que** le kit comportant ledit identifiant unique et ledit support d'enregistrement (7) est le kit d'instruments de pose.

7. - Procédé de traçabilité d'un système d'instrumentation chirurgicale constitué par un kit d'instruments à usage unique de pose d'implant et au moins un kit d'implants stériles, chacun desdits kits comprenant un identifiant, le procédé comprenant l'enregistrement d'un code du patient sur un support d'enregistrement (7) d'identifiants additionnels associé à l'un desdits kits comprenant un identifiant unique, ledit support étant constitué par une feuille contenue dans le kit comprenant l'identifiant unique, ladite feuille portant un identifiant reprenant l'identifiant unique dudit kit, ledit identifiant unique permettant l'identification du kit comprenant l'identifiant unique, le procédé comprenant de plus l'enregistrement en outre sur ledit support d'enregistrement (7) d'identifiants additionnels comprenant au moins un identifiant transférable d'au moins un kit complémentaire, au moment de l'utilisation dudit kit complémentaire.

8. - Procédé de traçabilité d'un système d'instrumentation chirurgicale selon la revendication 7 **caractérisé en ce qu'**il comporte une étape de transmission, à une base de donnée de collecte, dudit support d'enregistrement à l'issue de l'exploitation dudit système sur un patient, et **en ce qu'**il comporte une étape d'édition d'une fiche de synthèse à partir des données collectées au moins.

9. - Procédé de traçabilité d'un système d'instrumentation chirurgicale selon la revendication 7 **caractérisé en ce qu'**il comporte une étape de transmission à un collecteur de déchets à recycler d'une fiche de traçabilité regroupant les informations provenant du support d'enregistrement (7).

## Patentansprüche

1. Rückverfolgbarkeitssystem eines chirurgischen Instrumentensystems, wobei das System aus einem Einweg-Instrumentensatz zum Einbringen von Implantaten (1) und mindestens einem Satz steriler Implantate (2, 3) besteht, wobei jeder dieser Sätze eine Kennung aufweist,
der erste Satz (2, 3) eine einmalige Kennung (4), die die Identifizierung des Satzes mit einheitlicher Kennung ermöglicht, und der zweite Satz (3, 2) mindestens eine Kennung auf einem übertragbaren Informationsträger,
wobei der erste Satz einen Aufzeichnungsträger (7) für zusätzliche Kennungen aufweist, welcher aus einem Bogen mit einer Kennung besteht, die die einheitliche Kennung aufgreift,
wobei besagter Bogen im ersten Satz enthalten ist,
wobei eine der besagten zusätzlichen Kennungen ein Code im Zusammenhang mit dem Patienten sein kann, der während der Benutzung des besagten Satzes eingetragen wird,
wobei mindestens eine andere zusätzliche Kennung die Kennung auf einem übertragbaren Informationsträger (5, 6) des zweiten Satzes ist,
wobei besagte Kennung auf dem übertragbaren Informationsträger (5, 6) dazu bestimmt ist, zum Zeitpunkt der Benutzung des zweiten Satzes auf besagten Aufzeichnungsträger (7) für zusätzliche Kennungen übertragen zu werden.

2. Rückverfolgbarkeitssystem eines chirurgischen Instrumentensystems nach Anspruch 1, **dadurch gekennzeichnet, dass** besagte einheitliche Kennung (4) aus einem optisch erkennbaren Code besteht.

3. Rückverfolgbarkeitssystem eines chirurgischen Instrumentensystems nach Anspruch 1, **dadurch gekennzeichnet, dass** besagte einheitliche Kennung (4) aus einem Code besteht, der in einem elektronischen Speicher gespeichert wird.

4. Rückverfolgbarkeitssystem eines chirurgischen Instrumentensystems nach einem der vorhergehenden Ansprüche, **dadurch gekennzeichnet, dass** der Aufzeichnungsträger (7) für zusätzliche Kennungen des Weiteren Bereiche zur Aufnahme der Informationsträger der übertragbaren Kennungen und einen Bereich zur Aufnahme des Patientencodes aufweist.

5. Rückverfolgbarkeitssystem eines chirurgischen Instrumentensystems nach einem der vorhergehenden Ansprüche, **dadurch gekennzeichnet, dass** es des Weiteren eine Datenbank zur Speicherung der Informationen über die Herstellung, Verpackung und Sterilisierung der Sätze umfasst, wobei das System Mittel umfasst, um die vom Aufzeichnungsträger (7) für zusätzliche Kennungen stammenden Informationen ausgehend von der einheitlichen Kennung, die mit besagtem Träger verknüpft ist, zusammen zu tragen.

6. Rückverfolgbarkeitssystem eines chirurgischen Instrumentensystems nach einem der vorhergehenden Ansprüche, **dadurch gekennzeichnet, dass** der Satz, welcher besagte einheitliche Kennung und besagten Aufzeichnungsträger (7) für zusätzliche Kennungen umfasst, der Einbringinstrumentensatz ist.

7. Rückverfolgbarkeitssystem eines chirurgischen Instrumentensystems nach einem der vorhergehenden Ansprüche, bestehend aus einem Einweg-Instrumentensatz zum Einbringen von Implantaten und mindestens einem Satz steriler Implantate, wobei jeder dieser Sätze eine Kennung umfasst, wobei das Verfahren aus der Registrierung eines Patientencodes auf einem Aufzeichnungsträger (7) für zusätzliche Kennungen, der mit einem der besagten Sätze mit einheitlicher Kennung verknüpft ist, besteht, wobei besagter Informationsträger aus einem in einem Satz enthaltenen Bogen besteht, der die einheitliche Kennung aufweist, wobei besagter Bogen eine Kennung trägt, die die einheitliche Kennung des besagten Satzes aufgreift, wobei besagte einheitliche Kennung die Identifikation des Satzes ermöglicht, der die einheitliche Kennung aufweist, wobei das Verfahren des Weiteren in der Registrierung zusätzlicher Kennungen auf besagtem Aufzeichnungsträger (7) zum Zeitpunkt der Benutzung des zusätzlichen Satzes besteht, die mindestens eine übertragbare Kennung von mindestens einem zusätzlichen Satz umfasst.

8. Rückverfolgbarkeitsverfahren eines chirurgischen Instrumentensystems nach Anspruch 7, **dadurch gekennzeichnet, dass** es einen Schritt der Übertragung des besagten Aufzeichnungsträgers nach der Nutzung des besagten Systems an einem Patienten an eine Datensammlung enthält, und dadurch, dass es einen Schritt der Ausgabe eines Übersichtsbogens ausgehend von den gesammelten Daten umfasst.

9. Rückverfolgbarkeitsverfahrens eines chirurgischen Instrumentensystem nach Anspruch 7, **dadurch gekennzeichnet, dass** es einen Schritt der Übertragung eines Rückverfolgbarkeitsdatensatzes, welcher die vom Aufzeichnungsträger (7) stammenden Informationen enthält, an einen Sammler für zu recycelnde Abfälle umfasst.

## Claims

1. - A system for tracing a surgical instrumentation system, with said system consisting of a set of disposable instruments for fitting implants (1) and at least one set of sterile implants (2, 3), with each one of said sets comprising one identifier,
with a first one of said sets (2, 3) containing a unique identifier (4) enabling the identification of the set comprising the unique identifier, with the second set (3, 2) including at least one identifier on a transferable medium,
with the first set comprising a medium (7) for recording additional identifiers consisting of a sheet bearing an identifier taking up the unique identifier,
with said sheet being contained inside the first set,
with one of said additional identifiers possibly being a code associated with a patient, collected upon using said set,
with at least another additional identifier being the identifier on a transferable medium (5, 6) of the second set,
with said identifier on a transferable medium (5, 6) being intended to be transferred onto said additional identifiers recording medium (7) upon using the second set.

2. - A system for tracing a surgical instrumentation system according to claim 1, **characterized in that** said unique identifier (4) consists of an optically recognizable code.

3. - A system for tracing a surgical instrumentation system according to claim 1, **characterized in that** said unique identifier (4) consists of a code stored in an electronic memory.

4. - A system for tracing a surgical instrumentation system according to any one of the preceding claims, **characterized in that** the additional identifiers recording medium (7) further comprises areas for receiving the transferable identifiers media, and an area for receiving the patient code.

5. - A system for tracing a surgical instrumentation system according to any one of the preceding claims, **characterized in that** it further comprises a database for recording information relating to the manufacturing, packaging and sterilization of all the sets, with the system comprising means for reconciling the information from the additional identifiers recording medium (7) from the unique identifier associated with said medium.

6. - A system for tracing a surgical instrumentation system according to any one of the preceding claims, **characterized in that** the set comprising said unique identifier and said recording medium (7) is the fitting instrument set.

7. - A method for tracing a surgical instrumentation system consisting of a set of disposable instruments for fitting implants and at least one set of sterile implants, with each one of said sets comprising an identifier, with the method comprising the recording of the patient code on an additional identifiers recording medium (7) associated with one of said sets comprising a unique identifier, with said medium consisting in a sheet contained in the set comprising the unique identifier, with said sheet bearing one identifier taking up the unique identifier of said set, with said unique identifier enabling the identification of the set comprising the unique identifier,
with the method further comprising the further recording on said additional identifiers recording medium (7) comprising at least one transferable identifier of at least one complementary set upon using said complementary set.

8. - A method for tracing a surgical instrumentation system according to claim 7, **characterized in that** it comprises a step of transmitting, to a collection database, said recording medium upon completion of the operation of said system on a patient, and **in that** it comprises a step of editing a summary sheet at least from the data collected.

9. - A method for tracing a surgical instrumentation system according to claim 7, **characterized in that** it comprises a step of transmitting a traceability sheet gathering the information from the recording medium (7) to a collector of wastes to be recycled.
